# EUROPEAN PATENT SPECIFICATION

(11) **EP 0 866 137 B1**
(45) Date of publication and mention of the grant of the patent: **15.01.2003**
(21) Application number: 97301777.5
(22) Date of filing: 17.03.1997
(51) Int. Cl.: C12S 3/00, C07C 59/245

(54) **Process for producing calcium salt of (-)-Erythrohydroxycitric acid**
Verfahren zur Herstellung von Calcium (-)-Erythrohydroxyzitrat
Procédé de préparation de (-)-érythrohydroxycitrate de calcium

(43) Date of publication of application: 23.09.1998
(73) Proprietor: LUPIN LABORATORIES LIMITED, Mumbai, Maharashtra - 400098 (IN)
(72) Inventor: Sharma, Nina, Lupin Lab. Ltd. Nat. Pro. Div., Mumbai - 400 098, State Of Maharashtra (IN); Parashuraman, Meena, Lupin Lab. Ltd. Nat. Pro.Div., Mumbai - 400 098, State Of Maharashtra (IN); Raman, Girija, Lupin Lab. Ltd. Nat. Pro. Div., Mumbai - 400 098, State Of Maharashtra (IN)
(74) Representative: Harrison, David Christopher

(56) References cited:
- CHEMICAL ABSTRACTS, vol. 75, no. 14, 4 October 1971 Columbus, Ohio, US; abstract no. 92246, GLUSKER, JENNY P. ET AL: "Structure and absolute configuration of the calcium salt of garcinia acid, the lactone of (-)- hydroxycitric acid" XP002038799 & ACTA CRYSTALLOGR., SECT. B (1971), 27(PT. 7), 1284-93 CODEN: ACBCAR,
- CHEMICAL ABSTRACTS, vol. 71, no. 16, 20 October 1969 Columbus, Ohio, US; abstract no. 75298, GLUSKER, JENNY P. ET AL: "Absolute configurations of the naturally occurring hydroxycitric acids" XP002038800 & ARCH. BIOCHEM. BIOPHYS. (1969), 132(2), 573-5 CODEN: ABBIA4,
- CHEMICAL ABSTRACTS, vol. 124, no. 8, 19 February 1996 Columbus, Ohio, US; abstract no. 97375, SINGH, R.P. ET AL: "(-)- Hydroxycitric acid from Garcinia cambogia." XP002038801 & BIOL. MEM. (1995), VOLUME DATE 1995, 21(1), 27-33 CODEN: BMEMDK;ISSN: 0379-8097,
- CHEMICAL ABSTRACTS, vol. 97, no. 11, 13 September 1982 Columbus, Ohio, US; abstract no. 88720, KRISHNAMURTHY, N. ET AL: "Chemical constituents of kokam fruit rind" XP002038802 & J. FOOD SCI. TECHNOL. (1982), 19(3), 97-100 CODEN: JFSTAB;ISSN: 0022-1155,
- CHEMICAL ABSTRACTS, vol. 72, no. 17, 27 April 1970 Columbus, Ohio, US; abstract no. 89707, LEWIS, YOHAN SRIMANTH: "Isolation and properties of hydroxycitric acid" XP002038803 & METHODS ENZYMOL. (1969), 13, 613-19 CODEN: MENZAU,
- CHEMICAL ABSTRACTS, vol. 73, no. 1, 6 July 1970 Columbus, Ohio, US; abstract no. 3365, HORIKAWA, KAZUO ET AL: "Aliphatic hydroxycarboxylic acids as antioxidants. III. Preparation of two isomers of hydroxycitric acid lactone (erythro- and threo-3,4-dicarboxy-3-hydroxy-.gamma.-buty rolactone)" XP002038804 & BULL. CHEM. SOC. JAP. (1970), 43(2), 551-2 CODEN: BCSJA8, 1970,
- DATABASE WPI Section Ch, Week 9340 Derwent Publications Ltd., London, GB; Class A97, AN 93-316531 XP002038907 & JP 05 227 836 A (MEIJI SEIKA KAISHA) , 7 September 1993

## Description

The present invention relates to a process for the preparation of (-)-hydroxycitric acid as calcium salt from the fruit rinds of Garcinia species.

The calcium salt of (-)-hydroxycitric acid as well as (-)-hydroxycitric acid itself are therapeutically active components which alleviate fat formation. (-)-hydroxycitric acid is usually unstable in free form and gets converted to its corresponding lactone. The lactone of (-)-hydroxycitric acid is not therapeutically active since it shows little or no inhibition of deposit of fats [Ref. Clouatre et al, "The Diet and Health Benefits of hydroxycitric acid", A Good Health Guide pp 8-48 (1994)].

Accordingly to retain the therapeutic properties of (-)-hydroxycitric acid it was neutralised by alkali/alkaline agent e.g. calcium oxide, calcium hydroxide to its corresponding alkali or alkaline metal salts like sodium, potassium or calcium.

These salts of (-) erythro-hydroxycitric are suitable compounds possessing therapeutic properties of free hydroxycitric acid. (-) Erythro-hydroxycitric acid is the primary acid found in fruit rinds of Garcinia species such as Garcinia cambogia, Garcinia atroviridis and Garcinia indica. Garcinia species belong to the plant family known as Guttiferae. This species grow in the evergreen forests of Konkan stretching southwards in Kerala and Western Ghats and are useful in food preparations.

Usefulness of Garcinia species in food preparations is reported in literature. In the West Coast of South India, Garcinia Cambogia is used primarily in cooking and in Sri Lanka it is employed commercially in fish curing. G Indica and G, Atroviridis are also useful for culinary purposes especially in the preparation of curries. [ The Diet and Health Benefits of hydroxycitric acid, Clouatre et al, A Good Health Guide, pg. 8-9(1994); The Wealth of India, Vol. IV, p. 99, 100 CSIR New Delhi (1956); P.E.P. Deranlyagala, Ceylon J. Scl, Sec. (c) V, 49(1933); M F Chandratha, Trop. Agriculturist, 103,34(1947) ].

The concentration of (-)-hydroxycitric acid is found to be highest in Garcinia cambogia. Garcinia cambogia contains about 30% of (-)-hydroxycitric acid which is the biologically active component. (-)-Hydroxycitric acid was first isolated by Y.S Lewis and S Neelkanthan from the fruit rinds of G. cambogia and two other species and was characterized on the basis of chemical and spectroscopic studies [Y.S Lewis et al, Phytochemistry, Vol 4, p. 619 (1965) and Y.S. Lewis et al, Current Sci, Vol 33, p.82(1964)].

Since hydroxycitric acid (1,2-dihydroxypropane-1,2,3-tricarboxylic acid) has two asymmetric centres, four different isomers are possible of these (-)-erythro-hydroxycitric acid is biologically active and is isolated from the said Garcinia species. Hydroxycitric acid being a gamma-hydroxy acid, cyclises readily to the corresponding gamma-lactone. In nature the acid occurs in the form of its lactone which is different in chemical structure and physiological effect from the free acid, which inhibits fat formation. ["The Diet and Health Benefits of hydroxycitric acid, Clouatre et al, A Good Health Guide, pp, 8-9(1994)"]. (-)-Erythro-hydroxycitric acid is a therapeutically active molecule as an active inhibitor of fat formation [Lipids 9, 121-128(1971); ibid 12(1), 1-9(1977); ibid 12(4), 357-363(1977); ibid 9, 129-134(1974); Am. Journal of Clinical Nutrition 30(5), 777-784(1977); International Journal of obesity, 8, Supp 1, 241/248(1984)]. (-)-Erythro-hydroxycitric acid reduces fatty acid synthesis by inhibiting the enzyme ATP-citrate lyase which is responsible for converting excess of glucose into fat and cholesterol. It has also been shown to reduce the body weight and lower lipid accumulation in rats [Serglo W, Medical Hypothesls, 27 : 39(1988), Sullivan A.C. et al, Lipids, 9:121(1973) and Sullivan A.C. et al, Lipids, 9:129(1973)].

Various methods utilized in the prior art for the extraction of (-)-erythro-hydroxycitric acid are summarised below :
1. Y.S Lewis in Methods in Enzymology, 13:613(1967) described a process for the extraction of hydroxycitric acid wherein the acid was extracted using acetone or an alcohol such as ethanol and the resulting extract was treated with potassium hydroxide to yield potassium salt of the acid which was attempted to be converted to free hydroxycitric acid by passing through cation exchange resin. However, the attempt was futile since the acid was converted to its corresponding lactone and the yield of lactone was only 15%.
2. Indian Patent No. 160753 describes a process for the extraction of hydroxycitric acid along with garcinol and anthocyanins from rind of Garcinia indica wherein the extraction of fruit rind with acidulated water followed by treatment with alcohol yielded all the three desired products i.e. hydroxycitric acid, garcinol and anthocyanins which were separated by column chromatography. Hydroxycitric acid obtained by this process is unstable, cyclising to lactone and yield is only 16%.
3. WO 96/05741 describes a process for enriching hydroxycitric acid from Garcinia rind which comprises passing salt free water extract through anion exchange resin and liberating hydroxycitric acid in metal salt form by adding an alkali hydroxide such as sodium hydroxide. The hydroxycitrate salt is converted to its free acid by passing it through a cation exchange resin column wherein the free hydroxycitric acid, the lactone of hydroxycitric acid and citric acid constitute 94 to 99% by weight. of total solutes dissolved in water. However, this process utilising an ion exchange column would not be economical and also the total yield from the raw material is poor.
4. In addition to the above, the conventional process that is generally used in commerce consists of extraction of Garcinia cambogia fruit rind with water at 90°C to form the water extract. The residue remaining in the first extraction is further extracted twice in similar manner as the first extract to obtain two more additional extracts. Each extract after heating was combined and concentrated at 80°C till the volume was halved. The resulting concentrated extract was made alkaline by adding 20% aqueous sodium hydroxide solution till a pH of 4-5 is attained and kept overnight so that resinous pectin settles at the bottom. Next day the extract was decanted to remove the resinous matter and another portion of 20% aqueous solution of sodium hydroxide was added till it is alkaline (pH 7.5 to 8), followed by addition of aqueous calcium chloride till the pH of 6 to precipitate calcium salt of hydroxycitric acid. Methanol was added to enhance the precipitation process and the resulting calcium salt of hydroxycitric acid was filtered, dried to obtain light gray coloured product in 32.6% yield.

Most of the prior art processes relate to extraction of (-)-hydroxycitric acid, however, in most of the cases the attempts to isolate free (-)-hydroxycitric acid were futile, since the acid being unstable it slowly lactonises forming the corresponding lactone. Hence, free (-)-hydroxycitric acid is always associated with some amount of lactone.

The calcium salt of (-)-hydroxycitric acid is a commercial commodity and is manufactured in India by M/s. Lisalaya Herbals Ltd., Indore; M/s. Lucky Labs Ltd., New Delhi; M/s. Sharon Pharma Chem Ltd., Vashi, New Bombay and M/s. Hillgreen Company, Bangalore. The product is marketed in USA under the trade names "Citrin Garcinia Plus", "Citrimax", "Appe-Slim", "Pro-lean".

The conventional processes are carried out using number of steps and at elevated temperature which often present difficult operating conditions and result in high cost of production.

Thus the major object of the present invention is to solve the difficulties of the prior art and provide a simple, practically viable method for extraction of (-)-erythro-hydroxycitric acid from Garcinia fruit rinds.

A further object of the invention is to provide a process for extraction of (-)-erythro-hydroxycitric acid without necessarily using an organic solvent or excessive temperature.

Yet another object of the invention is to provide a process for the extraction of (-)-erythro-hydroxycitric acid without formation of its corresponding lactone.

The inventors have found that the catalytic use of a pectic enzyme such as polygalactouronase (PG) and pectin lysase (PL) or a mixture thereof is effective in inactivating structural components like pectins, cellulose and hemicellulose thereby making the extraction process of hydroxycitric acid more effective.

Thus the present invention relates to a process for producing calcium salt of (-)-erythro-hydroxycitric acid from fruit rinds of Garcinia species selected for example from Garcinia Cambogia, Garcinia indica and Garcinia atroviridis, the process comprising:
i) providing an aqueous suspension of fruit rinds of said Garcinia species;
ii) heating the aqueous suspension with a mixture of pectic enzymes at a temperature of between 30°C to 50°C for 1 to 3 hours;
iii) heating the mass to deactivate the enzyme(s);
iv) adding alkali to the resulting solution to achieve a pH of between 8-9;
v) adding calcium chloride to the product to precipitate the calcium salt of (-)-erythro-hydroxycitric acid.

The enzyme mixture is preferably used in the range of 0.0011 to 0.022 g per g of Garcinia fruit rind. Preferably according to the invention a mixture of pectic enzymes is used and such mixture of pectic enzymes consists of polygalactouronase (PG) and pectin lysase (PL) in the ratio of between 1:07 to 1:60 and preferably about 1:48. In the process of the invention the aqueous suspension of fruit rinds is provided by macerating fruit rinds of the Garcinia fruit rinds in water.

It is desirable that the mass obtained in step(ii) is filtered prior to the step of heating in step(iii) in which heating is effected at temperatures of about 70°C. The alkali used in the process is an aqueous alkaline solution and is chosen from sodium hydroxide, sodium carbonate, potassium chloride and potassium carbonate. The calcium salt of (-)-erythro-hydroxycitric acid thus obtained by the present process is dried.

According to preferred aspect of the present invention the process of the present invention for the extraction of (-)-ery-thro-hydroxycitric acid from Garcinia rind comprises macerating the Garcinia fruit rinds with water and 0.3% enzyme mixture of polygalactouronase (PG) and pectin lysase(PL) (1:60) at 40°C for two hours to yield the water extracts. The residue remaining in the first extraction is further extracted in similar manner to obtain two more extracts. Each extract was filtered and combined. The combined extracts were heated at 70°C for 1 hour to inactivate the enzymes to obtain a clear solution. To the resulting solution an aqueous alkali solution such as 20% sodium hydroxide solution was added till it is alkaline (pH 8-9). After cooling the reaction mixture to ambient temperature, 20% solution of calcium chloride was added and pH adjusted to 6-7 to precipitate out calcium salt of hydroxycitric acid. The resulting precipitate was filtered, washed and dried to obtain calcium salt of (-)-erythro-hydroxycitric acid in 51.89% yield based on the amount of Garcinia rind taken.

Garcinia fruit rinds contain hydroxycitric acid in its lactone form and some pectinous matter such as pectin, cellulose and hemicellulose. For therapeutic purpose hydroxycitric acid in its alkali or alkaline metal form should be free from the accompanying pectinous matter.

Pectin consists of polygalactouronic acid residues linked by beta 1,4 bonds and some of the carboxyl groups of the acid are present as methyl esters. The pectic enzyme, polygalactouronase (PG) hydrolytically cleaves the glycosidic bonds of polygalactouronic acids [Methods in Enzymology, Vol.1, pg. 158-166]. Another enzyme i.e. pectin lysase (PL) breaks the ester linkages of pectin, cellulose and hemicellulose.

The product obtained by the present process is lighter in colour 'as compared to that obtained using conventional process.

Total acids were estimated by titrimetry and were found to be 71%, of which hydroxycitric acid as analysed by HPLC was found to be 68.53%.

The mixture of pectic enzymes i.e. polygalactouronase (PG) and pectin lysase(PL) used in the present invention was obtained from M/s. Microzyme India Pvt. Ltd., Mumbai with the trade name Microlase.

The mixture of pectic enzymes i.e. polygalactouronase(PG) and pectin lysase (PL) was used in different combinations such as 1:07, 1:39, 1:48 and 1:60. However, the preferred combination of the said enzymes is 1:48, since high yield of calcium salt of hydroxycitric acid i.e. high hydroxycitric acid content was obtained when this combination was used.

The enzymes as used have a specific gravity of 1.1.

It is found that pectic enzymes play a major role in reducing the impurities such as pectin, cellulose and hemicellulose associated with hydroxycitric acid.

Not being bound by any theory it is believed that when added to the water extract of Garcinia rind the enzymes work by breaking the glycoside bonds of pectin, cleaving the polygalactouronic acids of pectin and breaking the ester linkages of pectin, cellulose and hemicellulose thereby enhancing release of (-)-erythro-hydroxycitric acid as compared to the prior art processes.

The ratio of pectic enzymes to substrate was found to be important. The higher the ratio of enzyme to the substrate, the faster is the release of hydroxycitric acid from the Garcinia rind. The results are presented in the following Table I.

**TABLE I**

| RATIO OF ENZYME TO SUBSTRATE w/w (%) | Ca SALT OF HCA % | HCA AS TOTAL ACIDS % |
|---|---|---|
| 0.042 | 17.30% | 55.46% |
| 0.091 | 36.37% | 70.39% |
| 0.110 | 51.89% | 71.00% |
| 2.200 | 57.20% | 72.80% |

The rate of extraction of hydroxycitric acid from Garcinia rind was studied in the presence and absence of enzymes at constant temperature of 40°C. It was observed that when the process was carried out in presence of enzymes the rate of release of hydroxycitric acid is faster. The results are presented in Figure I. The slow release of hydroxycitric acid in the absence of enzyme is evident from Figure I.

Also kinetics for extraction of hydroxycitric acid at elevated temperature of 90°C in the absence of enzymes was studied . It is evident from Figure I that even at higher temperature, the release of hydroxycitric acid is slow as compared to that obtained at 40°C in the presence of enzymes .

This data proves that the enzymes act as catalyst in effective release of hydroxycitric acid that is bound in Garcinia rinds.

The process of the present invention is carried out at a temperature ranging from 30°C to 50°C preferably at 40°C.

All attempts to isolate hydroxycitric acid at 40°C without enzymes, led to poor yield of the product which is evident from Figure I.

The time of the reaction depends on the conditions used i.e. temperature and composition and proportion of enzyme to the Garcinia rind and ranges from one to three hours depending on the parameters employed.

The process of the present invention can be carried out without use of any organic solvent, which is an improvement over the prior art.

The objects of the invention are illustrated by the following non-limiting examples:

### EXAMPLE 1:

### EXTRACTION USING MIXTURE OF PG AND PL (1:48)

Garcinia Cambogia rind (25.27 g) was taken in 100 ml water and 0.025 ml of pectic enzyme in mixture i.e. polygalactouronase(PG) and pectin lysase(PL) (1:48), was added, stirred and heated to 40°C for 3 hrs. The first extract was then filtered. The residue remaining in the first extraction was further extracted in similar manner as first extract to obtain two more additional extracts which were filtered and combined with the first extract. The combined extract was then heated at 70°C for 20 minutes to inactivate the catalytic enzymes to obtain extract rich in hydroxycitric acid. To the resulting solution 20% of aqueous sodium hydroxide was added to attain a pH of 8-9. The intermediate sodium salt was then treated with 20% aqueous calcium chloride to pH of 6 at which the calcium salt of hydroxycitric acid precipitates, which was dried at 90°C for 4 hours in a tray drier to yield 13.11 g (51.89%) calcium salt of hydroxycitric acid . The hydroxycitric acid content was 71.0% titrimetry.

### Optical Rotation

[free (-)-erythro-hydroxycitric acid] : [α]_{D}²⁰ = -20° (C = 10% in water)
[(-)-erythro-hydroxycitric acid : [α]_{D}²⁰ = -96° (C = 10% in water) saturated with borax]
I.R.(KBr) in cm -1 ( calcium salt of (-)-erythro-hydroxycitric acid ) : ° 3420, 1600, 1410.

### EXAMPLE 2:

### EXTRACTION USING MIXTURE OF PG AND PL (1:48)

Garcinia Cambogia rind (5 g) was taken in 25 ml water and 0.1 ml of pectic enzyme in mixture i.e. polygalactouronase(PG) and pectin lysase(PL) (1:48), was added and worked up in similar manner as in example 1. The yield of calcium salt of hydroxycitric acid obtained is 2.68 g (57.20%). The hydroxycitric acid content is 72.8% by titrimetry.

### EXAMPLE 3:

### EXTRACTION USING MIXTURE OF PG AND PL (1:48)

Garcinia Cambogia rind (24.12 g) was taken in 100 ml water and 0.02 ml of pectic enzyme in mixture i.e. polygalactouronase(PG) and pectin lysase(PL) (1:48), was added and worked up in a similar manner as in example 1. The yield of calcium salt of hydroxycitric acid obtained is 8.77 g (36.37%). The hydroxycitric acid content is 70.39% by titrimetry.

### EXAMPLE 4:

### EXTRACTION USING MIXTURE OF PG AND PL (1:48)

Garcinia Cambogia rind (26.15 g) was taken in 100 ml water and 0.01 ml of pectic enzyme in mixture i.e. polygalactouronase(PG) and pectin lysase(PL) (1:48), was added and worked up in a similar manner as in example 1. The yield of calcium salt of hydroxycitric acid obtained is 4.6 g (17.3%). The hydroxycitric acid content is 55.46% by titrimetry.

### EXAMPLE 5:

### EXTRACTION USING MIXTURE OF PG AND PL (1:07)

Garcinia cambogia rind (20.13 g) was taken in 100 ml water and 0.02 ml of pectic enzyme in mixture i.e. polygalactouronase(PG) and pectin lysase(PL) (1:07), was added and worked up in the same manner as in example 1. The yield of calcium salt of hydroxycitric acid obtained is 7.39 g (36.74%). The hydroxycitric acid content is 54.33% by titrimetry.

### EXAMPLE 6:

### EXTRACTION USING MIXTURE OF PG AND PL (1:38)

Garcinia Cambogia rind (19.94 g) was taken in 100 ml water and 0.02 ml of pectic enzyme mixture i.e. polygalactouronase(PG) and pectin lysase(PL) (1:38), was added and worked up in the same manner as in example 1. The calcium salt of hydroxycitric acid is obtained in 5.75 g (28.82%) yield. The hydroxycitric acid content is 54.12% by titrimetry.

### EXAMPLE 7:

### EXTRACTION USING MIXTURE OF PG AND PL (1:48)

Garcinia indica rind (25.28 g) was taken in 100 ml water and 0.025 ml of pectic enzyme in mixture i.e. polygalactouronase(PG) and pectin lysase(PL) (1:48), was added and worked up in similar manner as in example 1. The yield calcium salt of hydroxycitric acid obtained is 3.67 g (14.52%). The hydroxycitric acid content is 46.07% by titrimetry.

### EXAMPLE 8:

### STUDY OF EXTRACTION KINETICS :

Three experiments were carried out to study the rate of extraction of (-)-erythro hydroxycitric acid from Garcinia fruit rind in the presence or absence of pectic enzymes at temperature of 40°C and 90°C respectively.

The procedure was as follows:

Garcinia cambogia fruit rind (10 g) was taken in water (150 ml).
1. To the resulting aqueous suspension 0.01 ml of pectic enzyme mixture i.e. polygalactouronase (PG) and pectin lysase(PL) (1:48) was added, stirred and kept at 40°C in an incubator.
2. The resulting aqueous suspension without adding enzyme was kept at 40°C in an incubator.
3. The resulting aqueous suspension without adding enzyme was refluxed at 90°C.

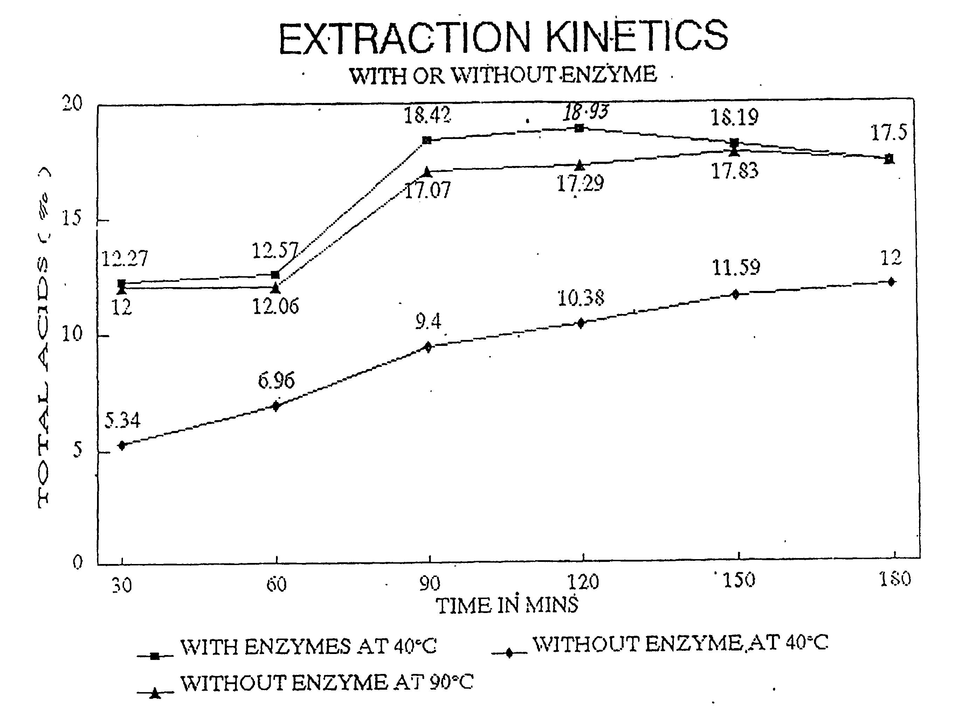

In each of the above cases 5 ml extract was withdrawn after every 30 minutes upto three hours and titrated against 0.1 N sodium hydroxide using phenolphthalein as an indicator. Depending on the consumption of sodium hydroxide the amount of total acid was calculated. The comparative data is presented in Table 2 and figure 1 respectively.

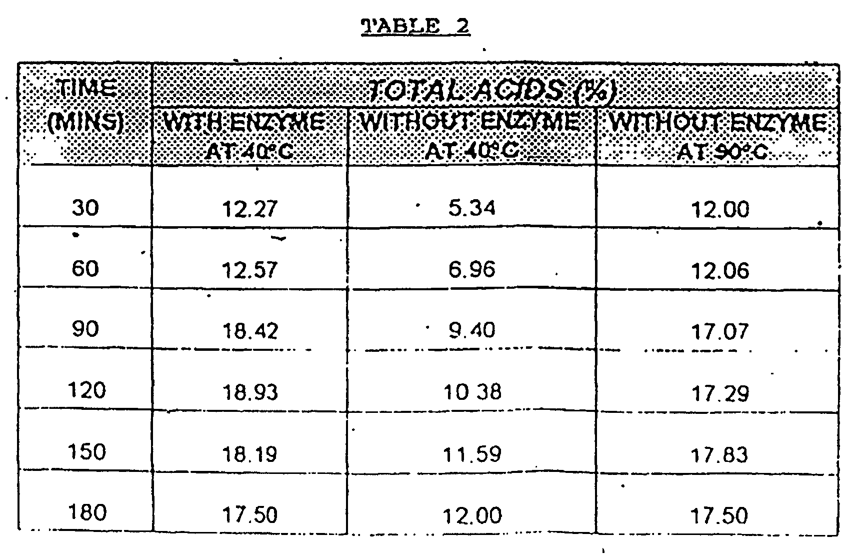

## Claims

1. A process for producing calcium salt of (-)-erythrohydroxycitric acid from fruit rinds of Garcinia species, the process comprising:
i) heating an aqueous suspension of fruit rinds of said Garcinia species with a mixture of pectic enzymes at a temperature of between 30°C to 50°C for 1 to 3 hours followed by separation of the rinds from the supernatant;
ii) heating the supernatant to deactivate the enzyme(s);
iii) adding alkali to the deactivated solution to achieve a pH of between 8-9;
iv) adding calcium chloride to the product to precipitate the calcium salt of (-)-erythrohydroxycitric acid.

2. A process as claimed in claim 1 wherein the Garcinia species is G. Cambogia, G. Indica or G. Atroviridia.

3. A process as claimed in claim 1 or claim 2 wherein said mixture of enzymes is used in the range of 0.0011 to 0.022 g per g of Garcinia fruit rind.

4. A process as claimed in any one of the preceding claims wherein the mixture of pectic enzymes consists of polygalactouronase (PG) and pectin lysase (PL) in a ratio of between 1:07 to 1:60.

5. A process as claimed in claim 4 wherein the ratio of polygalactouronase (PG) and pectin lysase (PL) is 1:48.

6. A process as claimed in any one of the preceding claims wherein the aqueous suspension of fruit rinds is provided by macerating fruit rinds of Garcinia fruit in water.

7. A process as claimed in any one of the preceding claims wherein the mass obtained in step (I) is filtered prior to the step of heating in step (ii).

8. A process as claimed in any one of the preceding claims wherein the temperature of heating in step (ii) is about 70°C.

9. A process as claimed in any one of the preceding claims wherein step (i) is carried out at a temperature of about 40°C.

## Patentansprüche

1. Verfahren zur Herstellung des Calciumsalzes von (-)-Erythrohydroxycitronensäure aus Fruchtrinden der Garcinia-Spezies, wobei das Verfahren Folgendes umfasst:
i) Erwärmen einer wässrigen Suspension von Fruchtrinden der Garcinia-Spezies zusammen mit einem Gemisch von Pektinenzymen auf eine Temperatur zwischen 30 °C und 50 °C für 1 bis 3 h, gefolgt vom Abtrennen der Rinden vom Überstand;
ii) Erhitzen des Überstands, um das/die Enzym(e) zu deaktivieren;
iii) Zusatz von Base zur deaktivierten Lösung, um einen pH zwischen 8 und 9 zu erzielen;
iv) Zusatz von Calciumchlorid zum Produkt, um das Calciumsalz von (-)-Erythrohydroxycitronensäure auszufällen.

2. Verfahren nach Anspruch 1, worin die Garcinia-Spezies G. Cambogia, G. Indica oder G. Atroviridia ist.

3. Verfahren nach Anspruch 1 oder 2, worin das Enzymgemisch im Bereich von 0,0011 bis 0,022 g pro g Carcinia-Fruchtrinde eingesetzt wird.

4. Verfahren nach einem der vorangegangenen Ansprüche, worin das Gemisch von Pektinenzymen aus Polygalacturonase (PG) und Pektin-Lysase (PL) in einem Verhältnis zwischen 1:07 und 1:60 besteht.

5. Verfahren nach Anspruch 4, worin das Verhältnis zwischen Polygalacturonase (PG) und Pektin-Lysase (PL) 1:48 beträgt.

6. Verfahren nach einem der vorangegangenen Ansprüche, worin die wässrige Fruchtrindensuspension durch Mazerieren von Fruchtrinden der Garcinia-Frucht in Wasser bereitgestellt wird.

7. Verfahren nach einem der vorangegangenen Ansprüche, worin die in Schritt (I) erhaltene Masse vor dem Schritt des Erhitzens in Schritt (ii) filtriert wird.

8. Verfahren nach einem der vorangegangenen Ansprüche, worin die Temperatur des Erhitzens in Schritt (ii) etwa 70 °C beträgt.

9. Verfahren nach einem der vorangegangenen Ansprüche, worin Schritt (i) bei einer Temperatur von etwa 40 °C durchgeführt wird.

## Revendications

1. Procédé de production du sel de calcium de l'acide (-)-érythrohydroxycitrique à partir de pelures de fruit de l'espèce Garcinia, le procédé comprenant:
i) le chauffage d'une suspension aqueuse des pelures de fruit de ladite espèce Garcinia avec un mélange d'enzymes pectiques à une température entre 30°C et 50°C pendant 1 à 3 heures avec ensuite séparation des pelures du produit surnageant;
ii) le chauffage du produit surnageant pour désactiver le(s) enzyme(s)
iii) l'addition d'un alcali à la solution désactivée pour obtenir un pH entre 8-9;
iv) l'addition de chlorure de calcium au produit pour précipiter le sel de calcium de l'acide (-)-érythrohydroxycitrique.

2. Procédé selon la revendication 1 où l'espèce Garcinia est G. Cambogia, G. Indica ou G. Atroviridia.

3. Procédé selon la revendication 1 ou la revendication 2 où ledit mélange d'enzymes est utilisé dans la gamme de 0,0011 à 0,022 g par g de pelures de fruit de Garcinia.

4. Procédé selon l'une quelconque des revendications précédentes où le mélange d'enzymes pectiques consiste en polygalactouronase (PG) et pectine lysase (PL) à un rapport entre 1:07 et 1:60.

5. Procédé selon la revendication 4 où le rapport de polygalactouronase (PG) et de pectine lysase (PL) est de 1:48.

6. Procédé selon l'une quelconque des revendications précédentes où la suspension aqueuse des pelures de fruit est obtenue par macération des pelures de fruit du fruit de Garcinia dans l'eau.

7. Procédé selon l'une quelconque des revendications précédentes où la masse obtenue à l'étape (I) est filtrée avant l'étape de chauffage à l'étape (ii).

8. Procédé selon l'une quelconque des revendications précédentes où la température du chauffage à l'étape (ii) est d'environ 70°C.

9. Procédé selon l'une quelconque des revendications précédentes où l'étape (i) est effectuée à une température d'environ 40°C.
